# EUROPEAN PATENT APPLICATION

(11) **EP 3 437 577 A1**
(43) Date of publication of application: **06.02.2019**
(21) Application number: 17184283.4
(22) Date of filing: 01.08.2017
(51) Int. Cl.: A61B 17/72

(54) **UNIVERSAL MODULAR BONE SEGMENT TRANSPORT DEVICE**

(71) Applicant: Medizinische Hochschule Hannover, 30625 Hannover (DE)
(72) Inventor: Krettek, Christian, 30173 Hannover (DE)
(74) Representative: Gramm, Lins & Partner Patent- und Rechtsanwälte PartGmbB

(57) **Abstract**

The present invention relates to a universal modular bone segment transport device which is completely implantable within a part of a body of a patient, wherein the bone segment transport device is capable of transporting at least one bone segment of the patient from a start position to a target position which is remote from the start position, wherein the bone segment transport device comprises at least the following:
a) a first fixation element, which is arranged for fixation on a first section of a bone of a patient,
b) a second fixation element, which is arranged for fixation on a second section of the bone of the patient,
c) a first transport element, which is arranged for fixation on a first bone segment which is located in an area between the second and the first section of the bone, wherein the first bone segment is to be transported by the bone segment transport device,
d) a first drive mechanism, which is arranged for effecting a motion of the first transport element relative to the first and/or second fixation element, characterized in that the second fixation element is a separate piece which is separate from the first fixation element, wherein the second fixation element can be coupled to the first fixation element for transportation of the first bone segment.

The device is universal, meaning that it can be attached to any commercially available lengthening nail. The device is modular, meaning it can be attached as a separate additional module if needed for segmental transport.

## Description

The present invention relates to the area of bone segment transportation of a patient. In case of defects of a bone, where a gap between a proximal and a distal section of the bone exists, the method of bone segment transport is very effective for filling the gap between the proximal and the distal section of the bone by newly formed bone regenerate. External fixation systems have already been used for fixation of the bone sections and transport of a bone segment. However, external fixation systems have disadvantages, like an increased risk of infection, pain, loss of range of motion in the adjacent joints and discomfort for the patient. It is therefore desirable to avoid or at least reduce these disadvantages by avoiding external fixation.

EP 2 570 092 A2 discloses a bone segment transport device which is completely implantable within a part of a body of a patient.

The object of the present invention is to provide for improved bone segment transport devices and their use.

This object is achieved by a bone segment transport device which is completely implantable within a part of a body of a patient, wherein the modular bone segment transport device is capable of transporting at least one bone segment of the patient from a start position to a target position which is remote from the start position, wherein the bone segment transport device comprises at least the following:
a) a first fixation element, which is arranged for fixation on a first section of a bone of a patient,
b) a second fixation element, which is arranged for fixation on a second section of the bone of the patient,
c) a first transport element, which is arranged for fixation on a first bone segment which is located in an area between the second and the first section of the bone, wherein the first bone segment is to be transported by the bone segment transport device,
d) a first drive mechanism, which is arranged for effecting a motion of the first transport element relative to the first and/or second fixation element,
characterized in a way that the second fixation element is a separate piece which is separate from the first fixation element, wherein the second fixation element can be coupled to the first fixation element for transportation of the first bone segment.

This allows for a complete implantation of the bone segment transport device, thus avoiding an increased risk of infection as it would occur with external systems. Also, any pain, restriction of joint movements and discomfort for the patient are reduced. In the start position, the first bone segment may abut on the first section of the bone. In the target position, the first transport bone segment may abut on the second section of the bone, such that the gap between the first section and the second section of the bone are now filled with newly built regenerative bone tissue. The first section of the bone may be a proximal section, the second section of the bone may be a distal section, or vice versa.

The bone segment transport device can be a modular bone segment transport device, e.g. a universal modular bone segment transport device which can be used with any commercially available bone lengthening nail as the second fixation element.

In contrast to prior art proposals, like EP 2 570 092 A2, the bone segment transport device comprises a second fixation element as a separate piece which can be coupled by the first fixation element if required, e.g. for the transportation purposes of the first bone segment. This also means that the second fixation element can be provided as an additional module for the bone segment transport device which can be chosen according to the specific situation. For example, different versions of the second fixation element with different length and/or diameter can be provided, such that the user can chose the most appropriate one of the available second fixation elements for the specific patient and transport situation for free combination with any available lengthening nail. The second fixation element can also be a separate piece which is separate from the first transport element.

The first drive mechanism can be an active or a passive drive mechanism. In case of an active drive mechanism, can comprise any kind of such drive mechanism, like electrically, magnetically or mechanically driven, or any combination thereof. In case of an electrically driven mechanism, the electric energy can be provided by a battery via wires, or contactless by inductive energy transfer. For example, the first drive mechanism can comprise an electric motor, for example with a gear mechanism and spindle, which can be provided as an actuator. The gear mechanism can be a planetary gear as this enables high transmission ratios. For the supply of power and control of the first drive mechanism a control unit can be provided that may be wirelessly supplied with energy and control signals via an antenna from outside the body in which the bone segment transport device is implanted. For example, the control unit can be able to wirelessly transmit or send captured parameters such as, for example, the force required for displacement or an already travelled displacement path. In case of a passive drive mechanism, the drive mechanism may comprise a shape-memory alloy and/or a spring for effecting the desired motion.

According to an advantageous embodiment the first fixation element comprises a first connection section, the second fixation element comprises a second connection section, wherein the first connection section is complementary to the second connection section, such that the second fixation element can be coupled to the first fixation element through the first and second connection section in a rigid or loose manner. This allows for a quick connection of the first fixation element to the second fixation element in a rigid or loose manner, for example, such that the second fixation element is inflexibly and in stiff way connected to the first fixation element. Torsional stability can be ensured through a torsionally stable fixation between first fixation element and the first transport element.

According to an advantageous embodiment the second connection section has at least one of a cone shape, a click-stop connector, or other form of connection like a jamming wire. The first connection section is then formed as a complementary connection element. The connection sections may be provided as a chuck cone for establishing a clamping connection between the first and the second fixation element.

According to an advantageous embodiment the first and/or the second fixation element comprises an inner cavity, the first transport element being longitudinally displaceable within the inner cavity. In such way, the first transport element can be located, at least in part, within the inner cavity and can move, in the course of transport of the first bone segment, longitudinally within this cavity. For example, the first and/or second fixation element can be formed like sleeve. The first fixation element can be made in one piece or as a separate element with the first drive mechanism. The second fixation element can made as a relatively simple mechanical part, like a sleeve with one or two elongated holes and one or more holes for fixation for the second section of the bone.

According to an advantageous embodiment a first attachment element for attaching the first bone segment to the first transport element extends through at least one elongated hole of the first and/or second fixation element. The first attachment element can be a bolt or a screw which extends in a transversal direction to the designated transport direction of the bone segment. The first attachment element penetrates, at least in part, in such direction the first bone segment and the first transport element. The elongated hole or elongated holes allow for the movement of the transport segment, together with the first bone segment, in the designated transport direction. In such way, the first attachment element in combination with the at least one elongated hole provides for a linear guiding of the first transport element and the first bone segment during their movement.

According to an advantageous embodiment the first fixation element, the first transport element and the first drive mechanism are parts of a commercially available bone lengthening nail, and the second fixation element is a generic universal supplementary module for the commercially available bone lengthening nail, for establishing, with or without further parts, the bone segment transport device. In such way, an already existing bone lengthening nail can be improved for use as bone segment transport device. This can be done by adding the second fixation element as a supplementary module. Since the second fixation element can be a very simple mechanical element, like a metal sleeve, the invention can be implemented with very little efforts.

According to an advantageous embodiment the bone segment transport device comprises a first engagement device, the first bone segment being connectable with a mounting section of the first transport element via the first engagement device, wherein the mounting section of the first transport element is ahead of the first bone segment in the designated transport direction of the first bone segment. This allows for use of the bone segment transport device in cases where the first bone segment is a very short segment with relatively small longitudinal dimension. In such cases, the first fixation element, first transport element and first drive mechanism can be still used without further modification, even in cases, when they are part of a bone lengthening nail.

The object of the invention is also achieved by a generic universal supplementary module for use with any commercially available bone lengthening nail as the second fixation element of a bone segment transport device according to any of the afore-mentioned embodiments. The generic universal supplementary module can be used with a bone lengthening nail with an active lengthening tube.

The first engagement device may allow for a fixed offset of the first transport element ahead of the first bone segment, or for a variable/adjustable offset which may be adjusted by the user upon the specific needs of the case.

The object of the invention is also achieved by a bone segment transport device for simultaneous transport of at least two bone segments of a patient, the bone segment transport device comprising at least the following:
a) a first fixation element, which is arranged for fixation on a first section of a bone of a patient,
b) a second fixation element, which is arranged for fixation on a second section of the bone of the patient,
c) a first transport element, which is arranged for fixation on a first bone segment which is located in an area between the second and the first section of the bone, wherein the first bone segment is to be transported by the bone segment transport device,
d) a first drive mechanism, which is arranged for effecting a motion of the first transport element relative to the first and/or second fixation element,
characterized by following features:
e) a second transport element, which is arranged for fixation on a second bone segment which is located in an area between the second and the first section of the bone, wherein the second bone segment is to be transported by the bone segment transport device,
f) a second drive mechanism, which is arranged for effecting a motion of the second transport element relative to the first and/or second fixation element.

By providing transportation and drive means for simultaneous transport of the at least two bone segments, the bone segment transport device allows for reducing the total time needed for filling the gap between the first and the second section of the bone to approximately the half time. This is possible without increasing the transport speed of each of the bone segments, but only by applying bone segment transport to at least two bone segments at the same bone in simultaneous manner.

The second transport element can be constructed in the same or a similar way like the first transport element. The second drive mechanism can be constructed in the same or a similar way like first drive mechanism. In particular, the second drive mechanism can be independent from the first drive mechanism, which allows for separate control of the movement and transport speed of the respective bone segments. The second transport element may be a simple fixation element, like a bolt or a screw, which allows for fixation of the second drive mechanism to the second bone segment.

The bone segment transport device may be implemented as an external system or as an internal system which is completely implantable within a part of a body of a patient.

According to an advantageous embodiment the second drive mechanism is a passive drive mechanism, which is coupled with the first drive mechanism, the second drive mechanism being drivable or driven by the first drive mechanism. In such way, the transport movement of the second transport element is directly dependent on the driving effect of the first drive mechanism. However, no further active elements like an electric motor and so on are required for establishing the second drive mechanism. For example, the second drive mechanism can be established by relatively simple mechanical means, like a towing cable and a reverse roller. In such case the reverse roller can be mounted to the first fixation element. The towing cable can be mounted with one end to the first transport element or the first bone segment, and with the other end to the second transport element or the second bone segment, thereby being wound around the reverse roller.

According to an advantageous embodiment the first and the second drive mechanisms are arranged for effecting opposite transport directions of the first and second bone segments. In this way, the first bone segment can be transported from the first section of the bone towards the second bone segment, and the second bone segment can be transported from the second section of the bone towards the first bone segment, until the first and the second bone segments converge.

The object of the invention is further achieved by the use of a bone segment transport device for a transport of at least one bone segment of a patient from a start position to a target position which is remote from the start position, and for lengthening of the same bone of the patient after the target position has been reached, resulting in additional lengthening of the bone if desired. In such way, the same bone segment transport device can be used for the mentioned two purposes, without additional surgery on the patient.

The object of the invention is further achieved by the use of a bone segment transport device of the afore-mentioned kind, wherein the bone segment transport device can be a bone segment transport device according to any of the afore-mentioned embodiments of such bone segment transport device.

The bone segment transport device is particularly suited for long hollow bones, like bones in the legs or arms of a patient.

The invention is further described using the following figures.
- Fig. 1: - first embodiment of a bone segment transport device;
- Fig. 2: - second embodiment of a bone segment transport device;
- Fig. 3: - third embodiment of a bone segment transport device;
- Fig. 4: - fourth embodiment of a bone segment transport device;
- Fig. 5: - two side views of a second fixation element;
- Fig. 6: - perspective view of the second fixation element of figure 5.

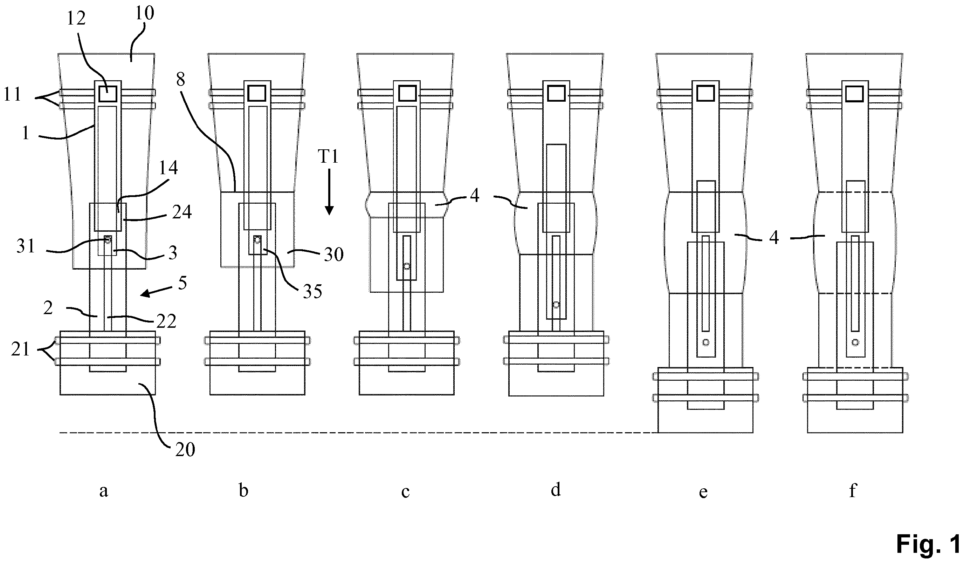

Figure 1 shows in a side view the first embodiment of the bone segment transport device and its use for filling a segmental defect of a bone. Further, the negative effects of a shortening of the bone are then reduced by a lengthening of a bone if needed.

The bone shown in figure 1 has a first section 10 and a second section 20. Between the first section 10 and the second section 20 there is a gap 5, which might be the result of an injury, infection or tumor. The gap 5, also called the segmental defect, shall be filled by newly generated bone tissue 4. For this purpose, the procedure of bone segment transport is used.

For conducting this bone segment transport, the bone segment transport device is first implanted in the bone structure. As can be seen in figure 1, a first fixation element 1 is implanted in the first section 10 of the bone. The first fixation element 1 is fixed to the first section 10 by means of one or more transversally inserted attachment elements 11, like screws or bolts. A second fixation element 2 is implanted in the second section 20 of the bone. The second fixation element 2 is fixed to the second section 20 in a similar way like the first fixation element, e.g. by means of attaching elements 21, like screws or bolts.

In its initial configuration after implantation, as depicted in picture a of figure 1, the second fixation element 2 is connected via its second connection section 24 to a first connection section 14 of the first fixation element 1. In this area, the first and second connection sections 14, 24 have an optional overlapping, which provides for additional secure and potentially rigid mechanical connection between the first and the second fixation element 1, 2.

In the initial configuration, the designated first bone segment for the later transport is still part of the first section 10. This designated first bone segment is fixed via a first attachment element 31, like a bolt, a screw or a wire or cable, to a mounting section 35 of a first transport element 3 of the bone segment transport device. The first transport element 3 can be established like a longitudinally displaceable piston which is guided, at least in part, within an inner hollow area of the first fixation element 1. The first transport element 3 is also guided within an inner cavity of the second fixation element 2. The second fixation element 2 comprises at least one or maybe two elongated holes 22, which allow for a guidance of the first attachment element 31 along the second fixation element 2 during the transport movement of the first bone segment 30.

As depicted in picture b), the first bone segment 30 is now separated from the first section 10 by an osteotomy 8. As shown in pictures c) and d), the transport of the first bone segment 30 from its start position (picture b) towards the second section 20 is now performed. The transport in the transport direction T1 is performed in very small steps, e.g. with a speed of 1 mm per day, for example with four steps of 0.25 mm per day. In the area between the first bone segment 30 and the first section 10 new bone tissue 4 is established by tissue regeneration. When the target position is reached, as depicted in picture d), the first bone segment 30 abuts on the second section 20. Therefore, the gap 5 has been filled. If no lengthening of the bone is required, the procedure can be stopped.

If a lengthening is required, the procedure can be continued until the desired lengthening is achieved, as depicted in pictures e, f.

The bone segment transport device comprises a first drive mechanism 12. The first drive mechanism 12 can be integrated, for example, into the first fixation element 1. The first drive mechanism 12 causes the motion for the transport of the first bone segment 30 and the lengthening of the bone.

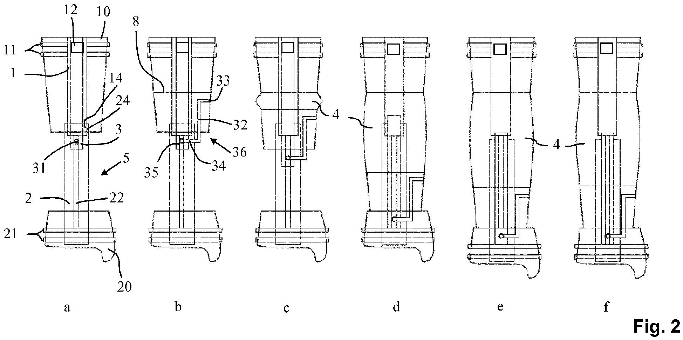

In some cases, the first section 10 or the second section 20 may be relatively short bone sections, with the effect that the longitudinal dimension of the bone segment transport device do not directly fit to the dimensions of the bone sections, which does not allow for a direct mechanical attachment of the first transport element 3 to the first bone segment 30 in the way as described in the first embodiment of the invention. As a solution for this issue, figure 2 shows a second embodiment of a bone segment transport device. In this embodiment, the bone segment transport device comprises a first engagement device 36 which provides for bridging the mounting section 35 of the first transport element 3 with the first bone segment 30. The first engagement device 36 comprises a mounting element 34 for mounting to the mounting section 35 of the first transport element 3, and a mounting element 33 for mounting to the first bone segment 30. The mounting elements 33, 34 are connected by a bridging element 32, which extends, at least in part, in the longitudinal direction, therefore, in the designated transport direction of the first bone segment 30. As can be seen, the mounting section 35 of the first transport element 3 is outside the first bone segment 30, in particular, it is ahead of the first bone segment 30 in the designated transport direction T1 of the first bone segment 30.

In this way, the second embodiment of the bone segment transport device, as depicted in figure 2, can be used for the same procedures as already explained in connection with figure 1, namely for filling the gap 5 and, if required, further for lengthening of the bone.

The first engagement device 36, in particular its bridging element 32, can be a rigid element, like a bar or a rod. This allows for transport of the first bone segment 30 in the designated transport direction T1. It further allows for corrections, namely for movements of the first transport element in the opposite direction of T1, e.g. for corrections of the procedure.

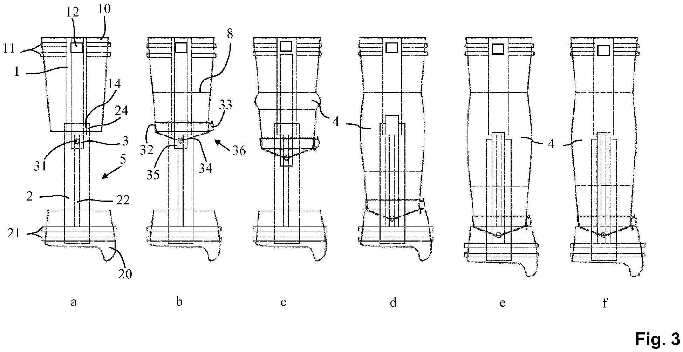

In the third embodiment of the bone segment transport device, as depicted in figure 3, there is also a first engagement device 36, which has similar functions as the first engagement device of the second embodiment. However, in the third embodiment the first engagement device 36 or at least its bridging elements 32 is not as rigid and stiff in the longitudinal direction (direction T1), such that it can only transfer towing force but not forces in the opposite direction of T1. This bridging element 32 can be, for example, a wire.

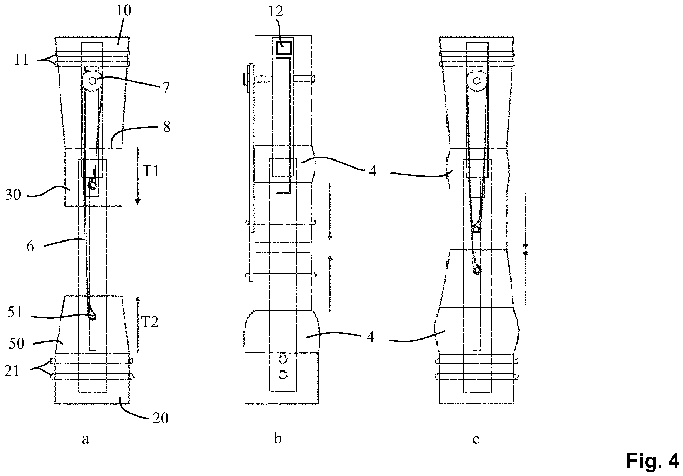

The fourth embodiment, as depicted in figure 4, is a bone segment transport device for simultaneous transport of two bone segments 30, 50. The bone segment transport device can be constructed in a similar or in the same way with regard to the first fixation element, the second fixation element, the first transport element and the first drive mechanism as it has been described for the afore-mentioned embodiments. For transporting the second bone segment 50, the bone segment transport device comprises a second transport element 51, which can be implemented, as depicted, in the form of a bolt or screw for fixation to the second bone segment.

However, in contrast to the embodiment shown in figure 4, which shows a second drive mechanism in the form of a passive drive mechanism, the second drive mechanism could also be an active drive mechanism, similar to the first drive mechanism 12. In such case the second transport element 51 can be similar to the first transport element, namely in the form of a piston-like element which can be moved by the second drive mechanism through the inner cavity of the second fixation element 2.

Getting back to the embodiment shown in figure 4, the second drive mechanism comprises a wire 6 which is connected with its one end to the second transport element 51 and with its other end to the mounting section 35 of the first transport element 3. Further, as a part of the second drive mechanism, a roller 7 is mounted on the first fixation element 1. The wire 6 is wound around the roller 7, in order to produce a movement in the direction T2 on the second bone segment 50, opposite to the transport direction T1 of the first bone segment 30. When the first transport element 3 is moved by the first drive mechanism 12, the same movement (in the opposite direction) is effected on the second transport element 51 by the second drive mechanism 6, 7.

In figure 4, picture a shows in a front view the initial situation after implantation of the bone segment transport device and after the osteotomy to separate the first bone segment 30 from the first section 10 and the second bone segment 50 from the second section 20. Picture b shows a side view, after some movement of the first and second bone segments 30, 50 have been induced. As can be seen, new bone tissue 4 is established between the first bone segment 30 and the first section 10, as well as between the second bone segment 50 and the second section 20. Picture c shows in a front view the final situation, when the first and second bone segments 30, 50 abut on each other.

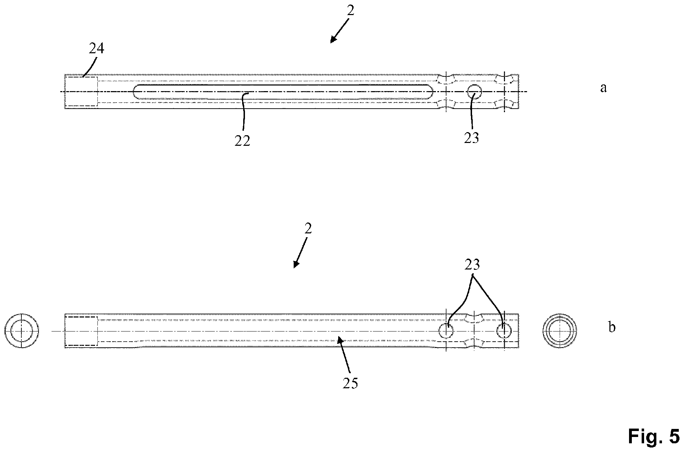

Figure 5 shows an embodiment of the second fixation element 2 in the form of a supplementary module for a bone lengthening nail. Figure 5 shows the second fixation element 2 in a front view (picture a) and in a side view (picture b). As can be seen, the second fixation element 2 is a relatively simple mechanical part in the form of a sleeve, e.g. a metal sleeve, which has an inner cavity. On one side or on two opposite sides the second fixation element 2 may comprise elongated holes 22, through which the first attachment element 31 may protrude. On the one end are one or more holes 23 for insertion of the attaching elements 21, in order to fixate the second fixation element 2 to the second section 20 of the bone. On the other end the second connection element 24 is depicted.

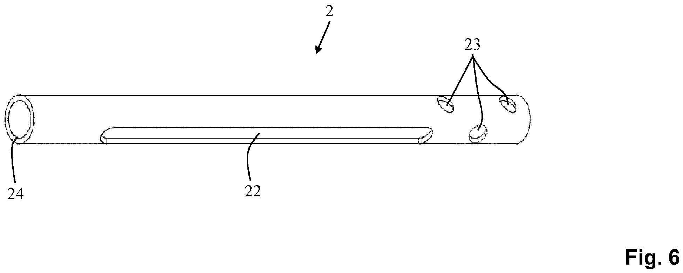

Figure 6 shows the second fixation element 2 of figure 5 in a perspective view.

## Claims

1. Bone segment transport device which is completely implantable within a part of a body of a patient, wherein the bone segment transport device is capable of transporting at least one bone segment (30, 50) of the patient from a start position to a target position which is remote from the start position, wherein the bone segment transport device comprises at least the following:
a) a first fixation element (1), which is arranged for fixation on a first section (10) of a bone of a patient,
b) a second fixation element (2), which is arranged for fixation on a second section (20) of the bone of the patient,
c) a first transport element (3), which is arranged for fixation on a first bone segment (30) which is located in an area between the first and the second section (10, 20) of the bone, wherein the first bone segment (30) is to be transported by the bone segment transport device,
d) a first drive mechanism (12), which is arranged for effecting a motion of the first transport element (3) relative to the first and/or second fixation element (1, 2),
**characterized in that** the second fixation element (2) is a separate piece which is separate from the first fixation element (1), wherein the second fixation element (2) can be coupled to the first fixation element (1) for transportation of the first bone segment (30).

2. Bone segment transport device according to claim 1, **characterized in that** the first fixation element (1) comprises a first connection section (14), the second fixation element (2) comprises a second connection section (24), wherein the first connection (14) section is complementary to the second connection section (24), such that the second fixation element (2) can be coupled to the first fixation element (1) through the first and second connection (14, 24) section in a rigid or loose manner.

3. Bone segment transport device according to claim 2, **characterized in that** the second connection section (24) has at least one of a cone shape, a click-stop connector, a jamming connection.

4. Bone segment transport device according to any of the preceding claims, **characterized in that** the first and/or the second fixation element (1, 2) comprises an inner cavity (25), the first transport element (3) being longitudinally displaceable within the inner cavity (25).

5. Bone segment transport device according to claim 4, **characterized in that** a first attachment element (31) for attaching the first bone segment (30) to the first transport element (3) extends through at least one elongated hole (22) of the first and/or second fixation element (12).

6. Bone segment transport device according to any of the preceding claims, **characterized in that** the first fixation element (1), the first transport element (3) and the first drive mechanism (12) are parts of a commercially available bone lengthening nail, and the second fixation element (2) is a generic universal supplementary module for the commercially available bone lengthening nail, for establishing, with or without further parts, the bone segment transport device.

7. Bone segment transport device according to any of the preceding claims, **characterized in that** the bone segment transport device comprises a first engagement device (36), the first bone segment (30) being connectable with a mounting section (35) of the first transport element (3) via the first engagement device (36), wherein the mounting section (35) of the first transport element (3) is ahead of the first bone segment (30) in the designated transport direction (T1) of the first bone segment.

8. Generic universal supplementary module for use with any commercially available bone lengthening nail as the second fixation element (2) of a bone segment transport device according to any of the preceding claims.

9. Generic universal supplementary module according to claim 8 for use with a bone lengthening nail with an active lengthening tube.

10. Bone segment transport device for simultaneous transport of at least two bone segments of a patient, the bone segment transport device comprising at least the following:
a) a first fixation element (1), which is arranged for fixation on a first section (10) of a bone of a patient,
b) a second fixation element (2), which is arranged for fixation on a second section (20) of the bone of the patient,
c) a first transport element (3), which is arranged for fixation on a first bone segment (30) which is located in an area between the first and the second section (10, 20) of the bone, wherein the first bone segment (30) is to be transported by the bone segment transport device,
d) a first drive mechanism (12), which is arranged for effecting a motion of the first transport element (3) relative to the first and/or second fixation element (1, 2),
**characterized by** following features:
e) a second transport element (51), which is arranged for fixation on a second bone segment (50) which is located in an area between the first and the second section (10, 20) of the bone, wherein the second bone segment (50) is to be transported by the bone segment transport device,
f) a second drive mechanism (6, 7), which is arranged for effecting a motion of the second transport element (51) relative to the first and/or second fixation element (1, 2).

11. Bone segment transport device according to claim 10, **characterized in that** the second drive mechanism (6, 7) is a passive drive mechanism, which is coupled with the first drive mechanism (12), the second drive mechanism (6, 7) being drivable or driven by the first drive mechanism (12).

12. Bone segment transport device according to claim 10 or 11, **characterized in that** the first and the second drive mechanisms (6, 7, 12) are arranged for effecting opposite transport directions (T1, T2) of the first and second bone segments (30, 50).

13. Bone segment transport device for use for transport of at least one bone segment of a patient from a start position to a target position which is remote from the start position and for lengthening of the same bone of the patient after the target position of the first bone segment (30) has been reached.

14. Bone segment transport device for use according to claim 13, **characterized in that** the bone segment transport device is a bone segment transport device according to any of claims 1 or 12.
